(19) Europäisches Patentamt
European Patent Office
Office européen des brevets

(11) Numéro de publication : **0 052 028**
**B1**

(12) **FASCICULE DE BREVET EUROPÉEN**

(45) Date de publication du fascicule du brevet : **08.02.84**

(51) Int. Cl.[3] : **C 07 C103/52**, A 61 K 37/02

(21) Numéro de dépôt : **81401600.2**

(22) Date de dépôt : **14.10.81**

(54) **Nouveaux hexapeptides, leur préparation, leur application comme médicaments et les compositions les renfermant.**

(30) Priorité : **14.10.80 FR 8021919**

(43) Date de publication de la demande : **19.05.82 Bulletin 82/20**

(45) Mention de la délivrance du brevet : **08.02.84 Bulletin 84/06**

(84) Etats contractants désignés : **AT BE CH DE GB IT LI LU NL**

(56) Documents cités :
**FR-A- 2 358 384**
**UNLISTED DRUGS, vol. 31, no. 11, 1979, 4e Edition Spec. Libr, Assoc.**

(73) Titulaire : **ROUSSEL-UCLAF**
**35, boulevard des Invalides**
**F-75007 Paris (FR)**

(72) Inventeur : **Gasc, Jean-Claude**
**6, Place Georges Lyssandre**
**F-93140-Bondy (FR)**
Inventeur : **Geoffre, Serge**
**11, Allée de la Garemotte Résidence Choisy Latour**
**F-33610-Cestas (FR)**
Inventeur : **Hospital, Michel**
**10, rue Professeur Roux**
**F-33400-Talence (FR)**
Inventeur : **Laurent, Jacques**
**63, rue du Fort**
**F-92130-Issy Les Moulineaux (FR)**

(74) Mandataire : **Niel, Michel et al**
**ROUSSEL-UCLAF boîte postale no 9 102, route de Noisy**
**F-93230 Romainville (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue St-Denis, 75001 Paris, France

# Nouveaux hexapeptides, leur préparation, leur application comme médicaments et les compositions les renfermant

La présente invention concerne de nouveaux hexapeptides, leur préparation, leur application comme médicaments et les compositions les renfermant.

La présente invention a pour objet les produits de formule générale (I)

Cys-X-Y-D-lys-Z (I)

dans laquelle X représente la séquence Ala-Ala ou la séquence Glu-His, Y représente Cys ou Phe et Z représente Phe lorsque Y représente Cys ou Cys lorsque Y représente Phe, les deux radicaux Cys présents dans la molécule étant reliés par un pont disulfure, ainsi que les dérivés fonctionnels desdits produits de formule (I).

Des peptides sont décrits dans le brevet français AKZO n° 2 358 384 mais ceux-ci ne renferment jamais de cystéine alors que le premier acide aminé des peptides de la présente demande est toujours une cystéine, reliée dans tous les cas à une deuxième cystéine par un pont di sulfure.

Unlisted Drug, volume 31, n° 11, novembre 1979, page 169, décrit un hexapeptide dont le premier ainsi que le dernier acide aminé sont représentés par une cystéine. Cependant, les 4 acides aminés entre ces deux cystéines sont différents de ceux constituant les peptides de la présente demande.

La nomenclature utilisée dans la présente demande est la nomenclature IUPAC, dont les règles sont publiées notamment dans Biochem. J. (1972) *126,* 773-780.

Conformément aux règles de cette nomenclature, les peptides désignés seulement par une abréviation à 3 lettres sont sous la forme L, existant naturellement. Lorsque ces peptides se présentent sous la forme D, la configuration (D) est précisée avant l'abréviation à 3 lettres.

Les symboles suivants ont donc été utilisés pour désigner les acides α-amino carboxyliques :

Cys : cystéine
Ala : alanine
Glu : acide glutamique
His : histidine
Phe : phenylalanine

Le terme « dérivés fonctionnels » de peptides de formule (I) peut désigner :

— des sels d'acides minéraux ou organiques, tels que, par exemple, ceux formés avec les acides chlorhydrique, bromhydrique, nitrique, sulfurique, phosphorique, acétique, trifluoroacétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques ;

— des sels de bases, de préférence une base dérivée d'un métal alcalin, telle que, par exemple, l'hydroxyde de sodium, le carbonate de sodium, ou le carbonate acide de sodium ;

— des esters d'alcoyle dont le radical alcoyle comporte de préférence de 1 à 5 atomes de carbone, comme, par exemple, les radicaux méthyl, éthyl ou propyl ;

— des amides primaires ou des amides alkyl substitués dans lesquels le ou les groupes alkyls renferment 1 à 5 atomes de carbone.

L'invention a notamment pour objet les produits tels que définis par la formule (I) ci-dessus, dans laquelle X représente la séquence Ala-Ala, ainsi que leurs dérivés fonctionnels, ainsi que les produits tels que définis par la formule (I) dans laquelle X représente la séquence Glu-His, ainsi que leurs dérivés fonctionnels.

Parmi ces produits, on retient plus particulièrement :

— le disulfure cyclique (1 → 6) de N-(cysteinylalanylalanyl phenylalanyl-D-lysyl) cystéine et son acétate,

— le disulfure cyclique (1 → 4) de N-(cysteinylalanylalanyl cysteinyl-D-lysyl) phénylalanine et son acétate,

— le disulfure cyclique (1 → 4) de N(cysteinylglutamyl histidylcysteinyl-D-lysyl) phénylalanine.

L'invention a également pour objet les complexes métalliques des peptides de formule (I) ou de leurs dérivés fonctionnels ci-dessus. Les complexes métalliques peuvent être préparés de façon connue et classique, en mettant les peptides au contact de sels métalliques (tels que par exemple les phosphates métalliques), d'hydroxydes métalliques ou d'oxydes métalliques. Les métaux utilisables sont par exemple le cobalt, le nickel, le cuivre, le fer et de préférence le zinc.

On peut préparer un complexe métallique par exemple en mélangeant le peptide et un sel, hydroxyde ou oxyde métallique en milieu aqueux.

Selon l'invention, les produits de formule (I) ci-dessus et leurs dérivés fonctionnels peuvent être préparés par un procédé, caractérisé en ce que l'on réalise des réactions de condensation d'acides α-amino carboxyliques successives dans l'ordre convenable, les acides α-amino carboxyliques étant protégés par des groupes de blocage qui sont ensuite éliminés, et que l'on transforme, si désiré, le produit de formule (I) obtenu en un dérivé fonctionnel.

Pour maîtriser de telles réactions, il est nécessaire de bloquer le groupe amino de l'un des acides et le groupe carboxylique de l'autre, et les groupes de blocage doivent pouvoir être facilement éliminés par la suite.

De nombreuses méthodes ont été mises au point pour la synthèse des peptides. Une des méthodes pouvant être utilisée est la technique de MERRIFIELD décrite dans JOURNAL OF AMERICAN. CHEMICAL SOCIETY *85,* pages 2149-2154, (1963).

Selon cette technique, on commence par lier un amino-acide à une particule de résine solide, par exemple par une liaison ester, et le peptide est produit en plusieurs étapes par fixation successives d'amino-acides protégés à la chaîne en développement. Les réactifs et les sous-produits sont éliminés par filtration.

Lorsque la séquence souhaitée a été assemblée, le peptide est enlevé du support solide.

Selon l'invention, les produits de formule (I) ci-dessus et les dérivés fonctionnels desdits produits peuvent être préparés selon un procédé de condensations successives d'amino-acides, dans lequel on utilise un support en phase solide.

Les amino-acides peuvent être attachés à tout polymère adapté qui doit simplement pouvoir facilement être séparé des réactifs n'ayant pas réagi. Il doit contenir un groupement fonctionnel auquel le premier amino-acide protégé peut être lié par une liaison covalente. Divers polymères conviennent pour cela, comme la cellulose, l'alcool polyvinylique, le polyméthacrylate, le polystyrène sulfoné par exemple. Lors de la préparation des produits donnés en exemple ci-après, on a utilisé une résine chlorométhylée de styrène et de divinylbenzène.

D'une manière générale, tous les groupes fonctionnels constitutifs peuvent être protégés d'une manière dont le principe est connu.

Les groupes amino en position alpha peuvent être protégés par exemple par les groupes t-butyloxycarbonyle, carbobenzoxy, adamantyloxycarbonyl ou isobornéyloxycarbonyle. On utilise essentiellement le groupe t-butyloxycarbonyle. Les amino-acides trifonctionnels peuvent être également protégés sur la fonction latérale. Pour la synthèse des différents produits objet de l'invention, on utilise Cys, Ala, Glu, His, D-lys. Le groupe SH de la cystéine peut être protégé par exemple par un groupe 4-méthoxybenzyl. Le groupe imidazole de l'histidine peut être protégé par exemple par un groupe 2,4-dinitrophényl. Le groupe gamma carboxyl de l'acide glutamique peut être protégé par un radical benzyloxy. Le groupe ε $NH_2$ de la D-lysine peut être protégé par un radical 2,4-dichlorobenzyloxycarbonyl.

Dans les exemples de synthèse donnés ci-après, le premier amino-acide est fixé sur la résine par l'intermédiaire de son sel de césium (cf B.F. GISIN, Helv. Chem. Acta vol *56,* fasc. 5, p. 1476 (1973)).

Les amino-acides ont été couplés par la méthode au dicyclohexylcarbodiimide. Les déblocages du radical butyloxycarbonyl ont été effectués par l'acide trifluoroacétique en solution à 50 % dans le chlorure de méthylène.

En fin de synthèse, on a éliminé le groupe de blocage 2-4, dinitrophényl de l'imidazol de l'histidine par le mercaptoéthanol. Le peptide obtenu a été séparé de la résine par l'acide fluorohydrique, ce qui a éliminé tous les autres blocages des fonctions latérales.

La transformation des produits de formule (I) en dérivés fonctionnels peut comprendre la formation des :

— sels formés par action des produits de formule (I) avec un acide tel que l'un de ceux mentionnés précédemment,

— sels formés par réaction des produits de formule (I) avec une base telle que l'hydroxyde de sodium, le carbonate acide de sodium, l'hydroxyde de potassium,

— esters, formés par réaction des produits de formule (I) avec un alcool tel que par exemple le méthanol, l'éthanol, le propanol, l'isopropanol, le butanol,

— amides, ou amides alkyl substitués dans lesquels le ou les groupes alkyls renferment de 1 à 5 atomes de carbone, selon des méthodes connues (cf. par exemple HOUBEN-WEYL, Methoden der organischen Chemie, 4[e] édition, vol. XVI, 1 pages 315 et suivantes).

Les tests à l'acide picrique donnant les titres d'amines libres ont été effectués d'après B.F. GISIN - Anal Chim. Acta *58,* 248 (1972).

Les analyses des amino-acides des peptides synthétisés ont été effectuées à l'aide d'un analyseur d'amino-acides BECKMAN.

Les produits de formule (I) ci-dessus, et les dérivés fonctionnels desdits produits de formule (I) possèdent d'intéressantes propriétés pharmacologiques ; à savoir que, notamment, ils retardent l'extinction de la réponse d'évitement conditionné, ils retardent la disparition de la réponse apprise, ils favorisent l'attention, la vigilance, la mémorisation.

Ces propriétés justifient leur application en thérapeutique, et l'invention a également pour objet, à titre de médicaments, les produits tels que définis par la formule (I) ci-dessus, ainsi que les dérivés fonctionnels pharmaceutiquement acceptables desdits produits de formule (I).

L'invention a ainsi notamment pour objet les médicaments, tels que définis ci-dessus, caractérisés en ce que dans la formule (I) X représente la séquence Ala-Ala, ainsi que leurs dérivés fonctionnels pharmaceutiquement acceptables, ainsi que ceux caractérisés en ce que dans la formule (I), X représente la séquence Glu-His, ainsi que leurs dérivés fonctionnels pharmaceutiquement acceptables.

L'invention a tout particulièrement pour objet, à titre de médicaments :

— le disulfure cyclique (1 → 6) de N-(cysteinylalanylalanylphénylalanyl-D-lysyl) cystéine et son acétate,
— le disulfure cyclique (1 → 4) de N-(cysteinylalanylcysteinyl-D-lysyl) phénylalanine et son acétate,
— le disulfure cyclique (1 → 4) de N-(cysteinylglutamylhistidylcysteinyl-D-lysyl) phénylalanine.

La présente demande a également pour objet, à titre de médicaments, les complexes métalliques pharmaceutiquement acceptables des peptides de formule (I) ou de leurs dérivés fonctionnels tels que définis ci-dessus.

Les produits de l'invention peuvent être utilisés par exemple dans le traitement des asthénies intellectuelles ou nerveuses, des défaillances de la mémoire, des troubles vasculaires cérébraux majeurs ou mineurs, de la sénescence ou du surmenage intellectuel.

Les produits de l'invention peuvent être administrés de préférence par voies intranasale, orale, parentérale, sublinguale ou rectale.

La dose usuelle est variable selon l'affection en cause, le produit utilisé, le sujet traité et la voie d'administration. La dose quotidienne peut être par exemple, par voie parentérale, de 0,01 μg à 500 μg par kilo de poids corporel, par les voies orale, intranasale et sublinguale de 0,1 mg à 500 mg par kilo de poids corporel.

L'invention s'étend aux compositions pharmaceutiques renfermant à titre de principe actif, l'un au moins des médicaments précités.

Ces compositions sont réalisées de façon à pouvoir être administrées par voies digestive, parentérale ou intranasale. Pour cela, on transforme les peptides en une forme convenant à l'administration par la voie choisie. De préférence, on les dissout, les met en suspension ou les émulsifie dans un liquide approprié. On peut également présenter ces peptides sous une forme convenant à l'administration orale, rectale, intranasale, parentérale ou sublinguale, en les mélangeant avec des excipients classiques. Ces compositions pharmaceutiques peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

On obtient des préparations particulièrement intéressantes en mettant les peptides de l'invention sous une forme où ils ont une activité prolongée, par exemple en les transformant en complexes métalliques, selon ce qui est indiqué plus haut.

Les exemples donnés ci-après illustrent l'utilisation de la technique en phase solide de MERRIFIELD, mais, comme il a été indiqué plus haut, d'autres techniques classiques de préparation des peptides peuvent être utilisées pour la préparation des produits objet de l'invention.

Les produits de l'invention peuvent par exemple être préparés en couplant des amino-acides isolés ou des peptides, convenablement protégés, à des amino-acides isolés ou à des peptides, également convenablement protégés, au moyen de substances activatrices de la fonction acide carboxylique, comme décrit dans Houben-Weyl : Methoden der organischen Chemie 15/2, Synthesen von Peptiden p. 2-364, (1974) Georg Thieme Verlag, Stuttgart, par exemple au moyen de dicyclohexylcarbodiimide, de N-éthyl-N'-(diméthylaminopropyl)-carbodiimide, d'o-nitrophénol, de p-nitrophénol ou de pentachlorophénol, avec ou sans addition de substances catalytiques.

Dans les exemples donnés ci-après les abréviations suivantes ont été employées :

| | |
|---|---|
| Boc = | t-butyloxycarbonyle |
| 4 MeO Bn = | méthoxybenzyl |
| 2,4-DNP = | 2,4 dinitrophényl |
| OBn = | benzyloxy |
| DMF = | diméthylformamide |
| DCC = | dicyclohexylcarbodiimide |
| HPLC = | chromatographie liquide préparative à haute performance |

Les exemples donnés ci-après illustrent l'invention sans toutefois la limiter.

Exemple 1

Acétate de disulfure cyclique (1 → 6) de N-/cysteinylalanylalanyl phénylalanyl-D-lysyl/cystéine

Peptide de formule : Cys-Ala-Ala-Phe-D-lys-Cys

Boc-(S-4-MeOBn) Cys-R (R = résine)

La fixation du premier amino-acide sur la résine est réalisée sur 10 g de résine de polystyrène à 1 % de divinylbenzène chlorométhylée (capacité de la résine : 0,87 meq par gramme de résine), dans 200 $cm^3$ de DMF, à l'aide de 4,73 g de sel de césium de la Boc S (4-MeOBn) L-cystéine (ledit sel de césium a été préparé en mélangeant de la Boc S (4-MeO Bn) L-cystéine, de l'éthanol, de l'eau et du carbonate de césium à pH = 7 en agitant à température ambiante pendant une heure). On agite 18 heures à 60 °C puis on essore la résine et lave avec trois fois 200 $cm^3$ de DMF, deux fois 200 $cm^3$ de méthanol, trois fois 200 $cm^3$ de chlorure de méthylène, puis sèche la résine sous vide et obtient 13 g de résine titrant 0,4 meq de L-cystéine bloquée par gramme de résine finale.

Cys-R

Les 13 g de la résine obtenue ci-dessus sont traités successivement par deux fois 150 $cm^3$ d'acide trifluoroacétique à 50 % dans le chlorure de méthylène pendant 10 minutes, deux fois 150 $cm^3$ de chlorure de méthylène, une fois 150 $cm^3$ d'isopropanol, deux fois 150 $cm^3$ de chlorure de méthylène, deux fois 150 $cm^3$ de triéthyl amine à 12 % de chlorure de méthylène pendant 10 minutes, deux fois 150 $cm^3$ de chlorure de méthylène, une fois 150 $cm^3$ d'isopropanol, une fois 150 $cm^3$ de méthanol, et six fois 150 $cm^3$ de chlorure de méthylène.

$N^{\alpha}$-Boc-$N^{\varepsilon}$ (2,4-dichloro benzyloxycarbonyl D-lysine-Cys-R

Sur Cys-R obtenu précédemment on ajoute 15,8 moles de $N^{\alpha}$-Boc-$N^{\varepsilon}$ (2,4-dichloro benzyloxycarbonyl) D-Lysine dissoutes dans 150 $cm^3$ de chlorure de méthylène, puis 18 $cm^3$ de DCC dans le chlorure de méthylène. Le couplage étant complet après 10 minutes (test à la ninhydrine négatif). On lave alors par 2 fois 150 $cm^3$ de chlorure de méthylène, 1 fois 150 $cm^3$ de méthanol, 1 fois 150 $cm^3$ d'isopropanol et 5 fois 150 $cm^3$ de chlorure de méthylène.

D-Lys-Cys-R

On débloque la D-Lys en opérant comme pour le déblocage de Cys ci-dessus.

Couplages suivants

On réalise ensuite les couplages, lavages, déblocages, comme pour les couplages, lavage, déblocage du deuxième amino-acide ci-dessus D-Lys, dans l'ordre suivant, avec les amino-acides protégés suivants :

- Boc L-phénylalanine
- Boc L-Alanine
- Boc L-Alanine
- Boc S-(4-méthoxybenzyl) L-Cystéine.

Après déblocage du dernier amino-acide, la cystéine, on obtient 16,8 g de résine finale.

Séparation du peptide de la résine

Dans un réacteur à acide fluorhydrique, on place les 16,8 g de résine obtenue précédemment, 18 $cm^3$ d'anisole, condense 80 $cm^3$ d'acide fluorhydrique, agite une heure à 0 °C, évapore sous vide, lave la résine avec 3 fois 200 $cm^3$ d'acétate d'éthyle, extrait alors le peptide Cys-Ala-Ala-Phe-D-Lys-Cys de cette résine avec successivement 4 fois 20 $cm^3$ d'acide acétique, 4 fois 20 $cm^3$ d'eau, 4 fois 20 $cm^3$ d'acide acétique à 50 % d'eau, réunit les extraits, lyophilise et obtient 3,9 g d'acétate de Cys-Ala-Ala-Phe-D-Lys-Cys. On dissout immédiatement le produit dans 100 $cm^3$ d'eau, filtre, recueille le filtrat qui est immédiatement utilisé pour l'étape suivante.

Cyclisation du peptide

Dans 500 $cm^3$ d'eau contenant 4 g de ferricyanure de potassium, on ajoute goutte à goutte en une heure trente minutes la solution de peptide obtenue précédemment en maintenant le pH à 7,5 avec de

l'ammoniaque 1 M. On passe ensuite la solution jaune obtenue sur 500 g de résine DOWEX ($Cl^-$), lave la résine par 2 fois 300 $cm^3$ d'eau bidistillée, lyophilise la solution incolore obtenue et obtient 6,6 g de

Cys-Ala-Ala-Phe-D-Lys-Cys brut.

Purification

On purifie le produit brut par chromatographie liquide préparative à haute performance (HPLC) sur cartouche de silice portant des chaînons hydrocarbonés en $C_{18}$, en éluant successivement :

1. avec 10 litres d'un mélange méthanol, eau bidistillée (15/85) contenant 3 g d'acétate d'ammonium par litre,

2. avec 2,5 litres d'un mélange méthanol-eau bidistillée 20/80 contenant 3 g d'acétate d'ammonium par litre.

On lyophilise le dernier éluant pour obtenir :

Cys-Ala-Ala-Phe-D-Lys-Cys,

reprend plusieurs fois avec de l'eau bidistillée, lyophilise jusqu'à poids constant, et obtient ainsi 1,91 g du peptide attendu purifié.

Analyse Amino-Acides

En théorie : Cys : Ala : Phe : Lys = 2 : 2 : 1 : 1
On obtient en fait = 2,02 : 2,12 : 1,02 : 1
$/\alpha/_D$(eau) = − 5°
Dichroïsme circulaire (eau)
max 220 nm $D_\varepsilon = +5{,}8$
max 270 nm $D_\varepsilon = -0{,}25$

## Exemple 2

Acétate de disulfure cyclique (1 → 4) de N-/cysteinylalanylalanylcystéinyl-D-lysyl/phénylalanine

Cys-Ala-Ala-Cys-D-lys-Phe $CH_3$ COOH
Boc-Phe-R (R = résine)

La fixation du premier amino acide sur la résine est réalisée sur 7,5 g de résine de polystyrène à 1 % de divinylbenzène identique à celle utilisée de la préparation du produit de l'exemple 1, à l'aide de 4,46 g de sel de césium : Boc-Phe-OCs, en agitant dans 95 $cm^3$ de DMF à 55 °C pendant une nuit.

Phe-R

Le groupement protecteur Boc est éliminé par action pendant 10 minutes d'acide trifluoroacétique en solution dans 50 % de chlorure de méthylène. La teneur en Phe de la résine, évaluée par le test à l'acide picrique est de 0,76 meq par gramme de résine. La résine est ensuite lavée avec successivement du DMF, du chlorure de méthylène, de l'isopropanol, en finissant par du chlorure de méthylène.

Boc-D-Lys-Phe-R

On réalise le couplage suivant de 4,49 g de $N^\alpha$-Boc-$N^\varepsilon$(2,4-dichlorobenzyloxy-carbonyl)-D-Lys-, à l'aide de 13 $cm^3$ d'une solution molaire de DCC dans le chlorure de méthylène.

La réaction de couplage est complète au bout d'une heure (le test à la ninhydrine étant alors négatif), on réalise des lavages successifs par 150 $cm^3$ de chlorure de méthylène, 150 $cm^3$ de méthanol, 150 $cm^3$ d'isopropanol et 5 fois 150 $cm^3$ de chlorure de méthylène.

On élimine le groupement Boc par action pendant 10 minutes de l'acide trifluoroacétique en solution dans 50 % de chlorure de méthylène, puis lave à nouveau par DMF, chlorure de méthylène, méthanol.

Boc (S-4 MeO Bn)-Cys-D-lys-Phe-R

On réalise ensuite le couplage suivant, en fixant 3,41 g de Boc-(S-4 MeOBn)-Cys dans 13 $cm^3$ d'une solution molaire de DCC dans le chlorure de méthylène.

Après une heure le test à la ninhydrine est négatif. On laisse ainsi sous agitation pendant 20 heures. On réalise les lavages, élimination de Boc et lavages comme après les deux couplages précédents.

Couplages suivants

On réalise ensuite les couplages, lavages, déblocages, lavages, comme pour les étapes précédentes, en utilisant dans l'ordre, les amino-acides protégés suivants :

— Boc-Ala (deux fois)
— Boc-(S-4-MeO Bn)-Cys, et obtient 14,5 g de résine, portant l'hexapeptide formé.

Cys-Ala-Ala-Cys-D-lys-Phe-R

Séparation du peptide de la résine (avec déprotection des chaînes latérales)

Les 14,5 g de résine obtenue ci-dessus sont hydrolysés par l'acide fluorhydrique dans un réacteur à acide fluorhydrique.

On place dans le réacteur 14,5 g de résine peptide, 14,5 $cm^3$ d'anisole, complète à 100 $cm^3$ d'acide fluorhydrique condensé à environ − 80 °C sous pression réduite, agite 45 minutes à 0 °C, évapore 90 minutes sous pression réduite, lave la résine à l'acétate d'éthyle avant d'extraire le peptide en lavant alternativement à l'eau distillée et à l'acide acétique, filtre, lyophilise le filtrat, et obtient 4,2 g d'acétate de Cys-Ala-Ala-Cys-D-lys-Phe

Cyclisation

On agite le peptide obtenu dans 350 $cm^3$ d'eau, filtre, verse le filtrat dans 450 $cm^3$ d'une solution 0,02 M de ferricyanure de potassium en maintenant le pH entre 6,5 et 7 par de l'ammoniaque 1M. On amène ensuite le pH à 5 par de l'acide acétique, agite la solution avec de la résine DOWEX ($Cl^-$), filtre, amène à sec à 25 °C sous pression réduite, reprend par de l'eau, lyophilise et obtient 6 g d'acétate de

Cys-Ala-Ala-Cys-D-lys-Phe.

Purification

Le produit brut est purifié par HPLC sur un appareil Prep 500 WATERS muni d'une cartouche de silice -$C_{18}$ en éluant les fractions contenant le produit successivement par des mélanges méthanol/eau contenant tous 3 g d'acétate d'ammonium par litre.

1. un mélange méthanol/eau (25 : 75)
2. un mélange méthanol/eau (21 : 79)
3. un mélange méthanol/eau (22 : 78)
4. un mélange méthanol/eau (23 : 77)

On obtient 0,365 g de produit pur attendu.

Analyse d'amino-acides :

en théorie :

Cys : Ala : Cys : Phe = 2 : 2 : 1 : 1
On obtient en fait : 1,96 : 2,06 : 1,08 : 1
$/\alpha/_D \simeq +16°$ (c = 0,5 %), (eau sur solution filtrée)
Dichroïsme circulaire (eau)
max 211 nm $D_\varepsilon = +4,7$
max 229 nm $D_\varepsilon = -6,0$
max 285 nm $D_\varepsilon = +0,3$

## Exemple 3

Disulfure cyclique (1 → 4) de N-/cysteinyl glutamylhistidylcysteinyl-D-lysyl/phénylalanine

Cys-Glu-His-Cys-D-lys-Phe
Boc-Phe-R (R = résine)

On utilise comme support solide 5 g d'une résine de polystyrène à 1 % de divinylbenzène chlorométhylée identique à celle utilisée pour la préparation du produit de l'exemple 1.

La condensation du premier amino-acide Phe est réalisée à l'aide de 2,978 g de Boc-Phe-OCs dissous dans 50 $cm^3$ de DMF. On chauffe à 50 °C sous agitation pendant 20 heures. Le déblocage de Phe est réalisé comme à l'exemple 1.

Couplages suivants

On réalise ensuite les couplages successifs, comme lors de la préparation du produit de l'exemple 1, en utilisant successivement les amino-acides protégés suivants :

— $N^{\alpha}$-Boc-$N^{\varepsilon}$(2,4-dichloro-benzyloxycarbonyl) D-Lys (on opère comme à l'exemple 1)
— Boc-(S-4-MeO Bn) Cys (on opère comme à l'exemple 1)
— α-Boc-(2,4 DNP) His (le groupement α-Boc est éliminé par action de l'acide trifluoracétique à 50 % dans le chlorure de méthylène et le groupement 2,4-dinitrophényl est éliminé en fin de synthèse par le 2-mercaptoéthanol comme indiqué ci-dessous :

— α Boc-Glu (O Bn)
— Boc-(S 4-MeO Bn) Cys

On obtient ainsi 10 g de Cys-Glu-(2,4 DNP) His-Cys-D-lys-Phe-R

Déblocage de 2,4 DNP de His

On mélange la totalité de la résine obtenue ci-dessus, 100 $cm^3$ de DMF, 100 $cm^3$ de 2-mercaptoéthanol, ajuste à pH = 8 avec de la triéthylamine, laisse sous agitation à température ambiante pendant 18 heures, essore la résine, lave au DMF, acétate d'éthyle, chlorure de méthylène, méthanol, et chlorure de méthylène, sèche sous pression réduite et obtient 9,75 g de l'hexapeptide attendu lié à la résine.

Séparation du peptide de la résine

On mélange 4,875 g du produit obtenu ci-dessus, 5 $cm^3$ d'anisole, 50 $cm^3$ d'acide fluorhydrique redistillé, sous agitation à 0 °C/ + 5 °C pendant 3/4 d'heure, élimine l'acide fluorhydrique et l'anisole sous pression réduite, lave la résine à l'acétate d'éthyle, puis récupère le produit en lavant à l'acide acétique pur puis à l'eau, filtre, lyophilise le filtrat, et obtient 2,143 g d'acétate de :

Cys-Glu-His-Cys-D-lys-Phe

Cyclisation

On opère comme à l'étape de cyclisation décrite ci-dessus à l'exemple 2, et obtient 1,704 g de produit attendu brut.

Purification

On purifie le produit brut sur carboxyméthylcellulose.
On dissout le produit brut dans une solution d'acétate d'ammonium 0,025 M à pH = 4 et fait passer cette solution sur la colonne en éluant avec une solution d'acétate d'ammonium 0,025 M à pH 4 puis une solution d'acétate d'ammonium 0,05 M à pH = 4.
Après lyophilisation on obtient :
422 mg de peptide attendu

Analyse d'amino-acides :

en théorie :

Cys : Glu : His : D-lys : Phe = 2 : 1 : 1 : 1 : 1
On obtient en fait :
1,6 : 1 : 1,02 : 1,12 : 1,12
$/\alpha/_D$ (c = 0,5 %, $H_2O$) = + 11° ± 2°
Dichroïsme circulaire (eau)
max ≃ 222 nm $D_\varepsilon$ ≃ − 2,8
max ≃ 260 nm $D_\varepsilon$ ≃ − 0,2

Etude pharmacologique

Action sur l'extinction d'un apprentissage d'évitement actif à renforcement négatif.
Le protocole et l'appareil utilisés ont été décrits par DE WIED (Proc. Soc. exp. Biol. Med. 1966, *122,* 28).
L'apprentissage est effectué dans une boîte équipée d'une lampe de 60 W (stimulus conditionnel : SC), d'un mât et d'un plancher électrifiable relié à un stimulateur électrique qui permet de délivrer un

choc douloureux de 75 volts (stimulus inconditionnel : SIC).

Le stimulus conditionnel (SC) est présenté seul pendant 5 secondes, puis est associé au stimulus inconditionnel (SIC) jusqu'à ce que l'animal s'aggripe au mât réalisant ainsi la réponse d'évitement recherchée. La réponse conditionnée (RC) est acquise lorsque l'animal répond au stimulus conditionnel.

Dix essais à 60 secondes d'intervalle sont effectués quotidiennement pendant 3 jours. L'examen de l'extinction de l'apprentissage réalisé avec suppression du stimulus inconditionnel (SIC) est suivi le 4ème jour au cours de trois essais à 2 heures d'intervalle sur les animaux présentant au moins 7 réponses conditionnées (RC).

Le composé est indiqué par voie sous cutanée sous forme de complexe phosphate-zinc aussitôt après le premier essai d'extinction de l'apprentissage à des lots de 5 rats. Les résultats sont comparés à ceux obtenus chez un lot d'animaux témoins.

L'évolution des réponses conditionnées au cours de la période d'extinction de l'apprentissage 2 et 4 heures après l'injection du composé est exprimée en pourcentage de variation par rapport au nombre de réponses conditionnées, observé au cours du premier essai d'extinction, avant traitement.

On a obtenu les résultats suivants :

| Produit administré | Dose µg/Rat | % de réponses conditionnées (RC) au cours de la période d'extinction de l'apprentissage | |
|---|---|---|---|
| | | après 2 heures | et 4 heures |
| Néant | | 60 | 30 |
| Produit de l'exemple 1 | 1 | 51 | 49 |
| | 10 | 76 | 71 |
| Produit de l'exemple 2 | 1 | 59 | 35 |
| | 10 | 71 | 75 |
| Produit de l'exemple 3 | 1 | 75 | 29 |
| | 10 | 70 | 50 |

Conclusion

On voit que par rapport aux résultats observés chez les animaux témoins, les produits de l'invention retardent l'extinction de l'apprentissage qui apparaît au cours du temps en l'absence de renforcement (le renforcement, qui est un renforcement négatif, étant constitué par le stimulus inconditionnel SIC).

**Revendications** (pour les Etats contractants : BE, CH, DE, GB, IT, LI, LU, NL)

1. Les produits de formule générale (I)

Cys-X-Y-D-lys-Z (I)

dans laquelle X représente la séquence Ala-Ala ou la séquence Glu-His, Y représente Cys ou Phe et Z représente Phe lorsque Y représente Cys ou Cys lorsque Y représente Phe, les deux radicaux Cys présents dans la molécule étant reliés par un pont disulfure, ainsi que les dérivés fonctionnels desdits produits de formule (I).

2. Les produits tels que définis par la formule (I) de la revendication 1, dans laquelle X représente la séquence Ala-Ala, ainsi que les dérivés fonctionnels desdits produits de formule (I).

3. Les produits tels que définis par la formule (I) de la revendication 1, dans laquelle X représente la séquence Glu-His, ainsi que les dérivés fonctionnels desdits produits de formule (I).

4. Le disulfure cyclique (1 → 6) de N-(cysteinylalanylalnyl phénylalanyl-D-lysyl) cystéine et son acétate.

5. Le disulfure cyclique (1 → 4) de N-(cysteinylalanylalanyl cysteinyl-D-lysyl) phénylalanine et son acétate.

6. Le disulfure cyclique (1 → 4) de N-(cysteinylglutamyl histidylcysteinyl-D-lysyl) phénylalanine.

7. Les complexes métalliques des peptides de formule (I) ou de leurs dérivés fonctionnels tels que définis à la revendication 1.

8. Procédé de préparation des produits de formule (I), tels que définis à l'une quelconque des revendications 1 à 6, caractérisé en ce que l'on réalise des réactions de condensation d'acides α-amino carboxyliques successives dans l'ordre convenable, les acides α-amino carboxyliques étant protégés par

des groupes de blocage qui sont ensuite éliminés, et que l'on transforme, si désiré, le produit de formule (I) obtenu en un dérivé fonctionnel.

9. Procédé de préparation selon la revendication 8, caractérisé en ce que l'on utilise un support en phase solide.

10. A titre de médicaments, les produits tels que définis par la formule (I) de la revendication 1, ainsi que les dérivés fonctionnels pharmaceutiquement acceptables desdits produits de formule (I).

11. A titre de médicaments, les produits selon les revendications 1 et 10, caractérisés en ce que dans la formule (I), X a la valeur indiquée à la revendication 2, ainsi que les dérivés fonctionnels pharmaceutiquement acceptables desdits produits de formule (I).

12. A titre de médicaments, les produits selon les revendications 1 et 10, caractérisés en ce que dans la formule (I), X a la valeur indiquée à la revendication 3, ainsi que les dérivés fonctionnels pharmaceutiquement acceptables desdits produits de formule (I).

13. A titre de médicament, l'un quelconque des produits définis aux revendications 4, 5 ou 6.

14. A titre de médicament, les complexes métalliques pharmaceutiquement acceptables définis à la revendication 7.

15. Compositions pharmaceutiques renfermant à titre de principe actif, l'un au moins des médicaments tels que définis à l'une quelconque des revendications 10 à 14.

**Revendications** (pour l'Etat contractant AT)

1. Procédé de préparation des produits de formule générale (I)

Cys-X-Y-D-lys-Z (I)

dans laquelle X représente la séquence Ala-Ala ou la séquence Glu-His, Y représente Cys ou Phe et Z représente Phe lorsque Y représente Cys ou Cys lorsque Y représente Phe, les deux radicaux Cys présents dans la molécule étant reliés par un pont disulfure, ainsi que des dérivés fonctionnels desdits produits de formule (I), des complexes métalliques des produits de formule (I) et des complexes métalliques de leurs dérivés fonctionnels, caractérisé en ce que l'on réalise des réactions de condensation d'acides α-amino carboxyliques successives dans l'ordre convenable, les acides α-amino carboxyliques étant protégés par des groupes de blocage qui sont ensuite éliminés, que l'on transforme, si désiré, le produit de formule (I) obtenu en un dérivé fonctionnel, et que l'on transforme, si désiré, le produit de formule (I) ou l'un de ses dérivés fonctionnels obtenu, en un complexe métallique.

2. Procédé de préparation selon la revendication 1, caractérisé en ce que l'on utilise un support en phase solide.

3. Procédé selon la revendication 1 ou 2, caractérisé en ce que X représente la séquence Ala-Ala.

4. Procédé selon la revendication 1 ou 2, caractérisé en ce que X représente la séquence Glu-His.

5. Procédé selon la revendication 1 ou 2, caractérisé en ce que l'on prépare l'un quelconque des produits de formule (I) dont les noms suivent :

— Le disulfure cyclique (1 → 6) de N-(cysteinylalanylalanyl phénylalanyl-D-lysyl) cystéine et son acétate.

— Le disulfure cyclique (1 → 4) de N-(cysteinylalanylalanyl cysteinyl-D-lysyl) phénylalanine et son acétate.

— Le disulfure cyclique (1 → 4) de N-(cysteinylglutamyl histidylcysteinyl-D-lysyl) phénylalanine.

**Claims** (for the Contracting States : BE, CH, DE, GB, IT, LI, LU, NL)

1. Products with the general formula (I)

Cys-X-Y-D-lys-Z (I)

in which X represents the Ala-Ala sequence or the Glu-His sequence, Y represents Cys or Phe and Z represents Phe when Y represents Cys or Cys when Y represents Phe, the two Cys radicals present in the molecule being linked by a disulphide bridge, as well as the functional derivatives of the said products with the formula (I).

2. Products as defined by formula (I) of Claim 1, in wgich X represents the Ala-Ala sequence, as well as the functional derivatives of the said products with the formula (I).

3. Products as defined by formula (I) of Claim 1, in which X represents the Glu-His sequence, as well as the functional derivatives of the said products with the formula (I).

4. The cyclic disulphide (1 → 6) of N(cysteinylalanylalanyl-phenylalanyl-D-lysyl)-cysteine and its acetate.

5. The cyclic disulphide (1 → 4) of N-(cysteinylalanylalanyl-cysteinyl-D-lysyl)-phenylalanine and its acetate.

6. The cyclic disulphide (1 → 4) of N-(cysteinylglutamylhistidylcysteinyl-D-lysyl)-phenylalanine.

7. The metal complexes of the peptides with the formula (I) of their functional derivatives as defined in Claim 1.

8. Preparation process for the products with the formula (I), as defined in any one of the Claims 1-6, characterized in that successive condensation reactions of α-amino carboxylic acids are carried out in a suitable order, the α-amino carboxylic acids being protected by blocking groups which are subsequently eliminated, and in that, if desired, the product with the formula (I) obtained is converted into a functional derivative.

9. Preparation process according to Claim 8, characterized in that a support in the solid phase is utilized.

10. As medicaments, the products as defined by formula (I) of Claim 1, as well as the pharmaceutically acceptable functional derivatives of the said products with the formula (I).

11. As medicaments, the products according to Claims 1 and 10, characterized in that in formula (I) X has the value indicated in Claim 2, as well as the pharmaceutically acceptable functional derivatives of the said products with the formula (I).

12. As medicaments, the products according to Claims 1 and 10, characterized in that in formula (I) X has the value indicated in Claim 3, as well as the pharmaceutically acceptable functional derivatives of the said products with the formula (I).

13. As medicament, any one of the products defined in Claims 4, 5 or 6.

14. As medicaments, the pharmaceutically acceptable metal complexes defined in Claim 7.

15. Pharmaceutical compositions containing as active principle at least one of the medicaments as defined in any one of the Claims 10-14.

**Claims** (for the Contracting State AT)

1. Preparation process for the products with the general formula (I)

Cys-X-Y-D-lys-Z (I)

in which X represents the Ala-Ala sequence or the Glu-His sequence, Y represents Cys or Phe and Z represents Phe when Y represents Cys or Cys when Y represents Phe, the two Cys radicals present in the molecule being linked by a disulphide bridge, as well as the functional derivatives of the said products with the formula (I), the metal complexes of products with the formula (I) and the metal complexes of their functional derivatives, characterized in that successive condensation reactions of α-amino carboxylic acids are carried out in a suitable order, the α-amino carboxylic acids being protected by blocking groups which are subsequently eliminated, in that, if desired, the product with the formula (I) obtained is converted into a functional derivative, and in that, if desired, the product with the formula (I) or one of its functional derivatives obtained is converted into a metal complex.

2. Preparation process according to Claim 1, characterized in that a support in the solid phase is utilized.

3. Process according to Claim 1 or 2, characterized in that X represents the Ala-Ala-sequence.

4. Process according to Claim 1 or 2, characterized in that X represents the Glu-His sequence.

5. Process according to claim 1 or 2, characterized in that any one of the products with the formula (I), named below, is prepared :

— the cyclic disulphide (1 → 6) of N-(cysteinylalanylalanylphenylalanyl-D-lysyl)-cysteine and its acetate,

— the cyclic disulphide (1 → 4) of N-(cysteinylalanylalanylcysteinyl-D-lysyl)-phenylalanine and its acetate,

— the cyclic disulphide (1 → 4) of N-(cysteinylglutamylhistidylcysteinyl-D-lysyl)-phenylalanine.

**Ansprüche** (für die Vertragsstaaten : BE, CH, DE, GB, IT, LI, LU, NL)

1. Die Produkte der allgemeinen Formel (I)

Cys-X-Y-D-Lys-Z (I)

worin X die Sequenz Ala-Ala oder die Sequenz Glu-His darstellt, Y Cys oder Phe bedeutet und Z Phe bedeutet, wenn Y Cys bedeutet oder Cys bedeutet, wenn Y Phe bedeutet, wobei die beiden in dem Molekül anwesenden Reste Cys über eine Disulfidbrücke gebunden sind sowie die funktionellen Derivate der Produkte der Formel (I).

2. Die Produkte der Formel (I) gemäß Anspruch 1, worin X die Sequenz Ala-Ala darstellt sowie die

funktionellen Derivate dieser Produkte der Formel (I).

3. Die Produkte der Formel (I) gemäß Anspruch 1, worin X die Sequenz Glu-His darstellt sowie die funktionellen Derivate dieser Produkte der Formel (I).

4. Cyclisches Disulfid (1 → 6) von N-(Cysteinylalanylalanylphenylalanyl-D-lysyl)-cystein und sein Acetat.

5. Cyclisches Disulfid (1 → 4) von N-(Cysteinylalanylalanylcysteinyl-D-lysyl)-phenylalanin and sein Acetat.

6. Cyclisches Disulfid (1 → 4) von N-(Cysteinylglutamylhistidylcysteinyl-D-lysyl)-phenylalanin.

7. Metallkomplexe der Peptide der Formel (I) oder von deren funktionellen Derivaten gemäß Anspruch 1.

8. Verfahren zur Herstellung von Produkten der Formel (I) gemäß einem der Ansprüche 1 bis 6, dadurch gekennzeichnet, daß man aufeinanderfolgende Kondensationsreaktionen von α-Aminocarbonsäuren in geeigneter Reihenfolge durchführt, wobei die α-Aminocarbonsäuren durch Blockierungsgruppen geschützt sind, die darauf entfernt werden, und daß man gewünschtenfalls das erhaltene Produkt der Formel (I) in ein funktionelles Derivat überführt.

9. Verfahren gemäß Anspruch 8, dadurch gekennzeichnet, daß man einen Träger in fester Phase verwendet.

10. Als Arzneimittel, die Produkte der Formel (I) gemäß Anspruch 1 sowie die pharmazeutisch verträglichen funktionellen Derivate dieser Produkte der Formel (I).

11. Als Arzneimittel, die Produkte gemäß den Ansprüchen 1 und 10, dadurch gekennzeichnet, daß in der Formel (I) X die in Anspruch 2 angegebene Bedeutung besitzt sowie die pharmazeutisch verträglichen funktionellen Derivate dieser Produkte der Formel (I).

12. Als Arzneimittel, die Produkte gemäß den Ansprüchen 1 und 10, dadurch gekennzeichnet, daß in der Formel (I) X die in Anspruch 3 angegebene Bedeutung besitzt sowie die pharmazeutisch verträglichen funktionellen Derivate dieser Produkte der Formel (I).

13. Als Arzneimittel, eines der in den Ansprüchen 4, 5 oder 6 definierten Produkte.

14. Als Arzneimittel, die pharmazeutisch verträglichen Metallkomplexe gemäß Anspruch 7.

15. Pharmazeutische Zusammensetzungen enthaltend als Wirkstoff, zumindest eines der Arzneimittel gemäß einem der Ansprüche 10 bis 14.

**Ansprüche** (für den Vertragsstaat AT)

1. Verfahren zur Herstellung der Produkte der allgemeinen Formel (I)

$$\text{Cys-X-Y-D-Lys-Z} \qquad (I)$$

worin X die Sequenz Ala-Ala oder die Sequenz Glu-His darstellt, Y Cys oder Phe bedeutet und Z Phe bedeutet, wenn Y Cys bedeutet oder Cys bedeutet, wenn Y Phe bedeutet, wobei die beiden in dem Molekül anwesenden Reste Cys über eine Disulfidbrücke gebunden sind sowie die funktionellen Derivate dieser Produkte der Formel (I), die Metallkomplexe der Produkte der Formel (I) und die Metallkomplexe ihrer funktionellen Derivate, dadurch gekennzeichnet, daß man aufeinanderfolgende Kondensationsreaktionen von α-Aminocarbonsäuren in geeigneter Reihenfolge durchführt, wobei die α-Aminocarbonsäuren durch Blockierungsgruppen geschützt sind, die darauf entfernt werden, daß man gewünschtenfalls das erhaltene Produkt der Formel (I) in ein funktionelles Derivat überführt, und daß man gewünschtenfalls das erhaltene Produkt der Formel (I) oder eines funktionellen Derivate in einen Metallkomplex überführt.

2. Verfahren gemäß Anspruch 1, dadurch gekennzeichnet, daß man einen Träger in fester Phase verwendet.

3. Verfahren gemäß Anspruch 1 oder 2, dadurch gekennzeichnet, daß X die Sequenz Ala-Ala darstellt.

4. Verfahren gemäß Anspruch 1 oder 2, dadurch gekennzeichnet, daß X die Sequenz Glu-His darstellt.

5. Verfahren gemäß Anspruch 1 oder 2, dadurch gekennzeichnet, daß man eines der Produkte der Formel (I) mit den folgenden Bezeichnungen herstellt :

— Cyclisches Disulfid (1 → 6) von N-(Cysteinylalanylalanylphenylalanyl-D-lysyl)-cystein und sein Acetat,

— Cyclisches Disulfid (1 → 4) von N-(Cysteinylalanylalanylcysteinyl-D-lysyl)-phenylalanin und sein Acetat,

— Cyclisches Disulfid (1 → 4) von N-(Cysteinylglutamylhistidylcysteinyl-D-lysyl)-phenylalanin.